Europäisches Patentamt

**European Patent Office**

**Office européen des brevets**

(11) Numéro de publication: **0 088 686**
A1

## (12) DEMANDE DE BREVET EUROPEEN

(21) Numéro de dépôt: **83400439.2**

(22) Date de dépôt: **04.03.83**

(51) Int. Cl.³: **A 23 J 1/00**, C 12 C 5/00,
C 13 D 1/08, C 13 L 3/00

---

(30) Priorité: **05.03.82 FR 8203765**
**05.03.82 FR 8203767**

(71) Demandeur: **SOCIETE DE VALORISATION AGROBIOLOGIQUE, Place Billard de Vaux, F-53300 Ambrières Les Vallées (FR)**
Demandeur: **PROTEINOL, Bassin no. 3 R.N. 11, F-17308 Rochefort Cedex (FR)**

(43) Date de publication de la demande: **14.09.83**
**Bulletin 83/37**

(72) Inventeur: **Bonamici, Ferdinand, 17, rue de la Courtille, F-53300 Ambrieres les Vallees (FR)**

(84) Etats contractants désignés: **AT BE CH DE GB IT LI LU NL SE**

(74) Mandataire: **Hufénus, Christiane et al, CABINET BEAU DE LOMENIE 55, rue d'Amsterdam, F-75008 Paris (FR)**

---

(54) **Procédé de traitement de matières végétales protéiques et glucidiques et de sous-produits végétaux humides dans une même installation.**

(57) Procédé de traitement des matières végétales protéiques et glucidiques et des sous-produits végétaux humides des industries agro-alimentaires dans une même installation, pour récupérer d'une part tout ou partie des constituants sous forme de tourteaux déshydratés, et d'autre part des jus glucidiques pour la fermentation et la destillation, comprenant essentiellement un broyage très fin de la matière première, un traitement physico-chimique des matières glucidiques et une séparation très poussée des phases soluble et insoluble, de façon à faire passer le maximum d'eau dans les jus pour éliminer ensuite cette eau au moindre coût énergétique par recompression mécanique des vapeurs.

Le procédé de traitement physico-chimique des plantes glucidiques permettant d'améliorer la séparation ultérieure des phases solide et liquide et le rendement d'extraction des glucides, comprend l'incorporation de fibres d'origine végétale (jusqu'à 10%) finement broyée à la plante également finement broyée, l'addition d'un acide pour amener le pH entre 1,5 et 4, le chauffage à 50 à 85°C pendant 30 minutes à 2 heures et la neutralisation ultérieure du mélange réactionnel.

Procédé de traitement de matières végétales protéiques et glucidiques et de sous-produits végétaux humides dans une même installation.

La présente invention se rapporte à un procédé permettant d'obtenir, à partir de matières premières végétales protéiques ou glucidiques ou de sous-produits végétaux contenant de 60 à 95 % d'eau, tels que des sous-produits des industries agro-alimentaires, une séparation suffisamment efficace et poussée des constituants solubles et insolubles pour permettre un fonctionnement global d'installation en énergie totale, ou nécessitant l'apport éventuel d'une faible quantité d'énergie thermique en plus de l'énergie électrique, ou même permettant un fonctionnement en tout électrique. Les installations pouvant fonctionner dans de telles conditions peuvent :

- soit, réaliser seulement la conservation des constituants alimentaires des plantes par élimination suffisante de l'eau ;
- soit, réaliser la conservation de certains des constituants et produire par ailleurs des alcools ou produits chimiques issus de fermentations adaptées.

On appellera ci-après :

- <u>Plantes protéiques</u> : les végétaux verts, apportant principalement des protéines dans l'alimentation ; par exemple, les légumineuses telles que luzerne et trèfle, et les graminées telles que ray grass, fétuque, etc.

- <u>Plantes glucidiques</u> : les racines et tubercules riches en glucides ; par exemple, topinambours, betteraves sucrières ou demi-sucrières ou fourragères, sorgho sucrier, etc.

- <u>Le rapport R</u> : Celui obtenu en divisant la consommation d'énergie primaire d'origine électrique par la consommation totale d'énergie primaire, ces consommations se rapportant au passage d'une siccité de 20 % matière sèche à 88 % matière sèche.

- <u>La consommation globale d'énergie G en thermies/tonne</u> : correspond à l'énergie primaire totale nécessaire pour réaliser le procédé de transformation de la matière première brute en produit sec avec granulation, ramenée à la tonne de matière sèche traitée. Dans le cas d'une distillerie, G inclut la consommation d'énergie de la distillation sans concentration des vinasses. On considère que l'électricité n'est pas produite à l'usine.

- <u>Phase insoluble ou tourteau</u> : Elle est constituée par la partie des plantes qui est insoluble dans l'eau de constitution de la plante ou dans l'eau de dilution. Dans le cas où un traitement chimique de la matière est requis, la phase insoluble est la phase résiduelle des constituants insolubles avant traitement chimique. Lors de la séparation des phases, soluble et insoluble, la phase insoluble contiendra la partie de phase soluble existant dans l'eau du tourteau. Plus la séparation des phases soluble et insoluble est efficace, plus la siccité du tourteau est élevée.

- <u>Phase soluble ou jus</u> : La phase soluble est la phase complémentaire à la phase insoluble, dans le processus de séparation des phases. La phase soluble contient une très faible partie de matières insolubles en suspension, soit au maximum 1 % de matière sèche par rapport à la matière sèche totale contenue dans la phase soluble. Plus la qualité de filtration est élevée, plus le pourcentage de matières insolubles dans les jus est réduit.

On rappellera succinctement les procédés existant à l'heure actuelle :

- La déshydratation classique, en circuit ouvert avec condensation partielle de l'eau évaporée, consiste à sécher dans un courant d'air chaud les produits hachés fins. La consommation énergétique est très élevée, le rapport R est de l'ordre de 0,18 et l'énergie globale G consommée est de 2.350 th/T. La déshydratation classique ne permet pas un traitement séparé des constituants de la plante traitée, le séchage étant effectué sur la plante, sans extraction préalable de jus.

- Le procédé de déshydratation décrit dans les brevets français n°2294647 et 2.302.048, conduit à une extraction partielle des matières solubles, les tourteaux obtenus à partir des plantes protéiques ayant une siccité de l'ordre de 25 à 30 % de matière sèche. Ce procédé permet d'obtenir des concentrés riches en protéines et vitamines, mais nécessite encore une forte consommation d'énergie, en particulier pour le séchage des tourteaux. Le rapport R est de l'ordre de 0,185 et G est de l'ordre de 2.300 th/T.

- En distillerie de betteraves sucrières modernes,

3

l'extraction des jus s'effectue par diffusion, les pulpes obtenues ayant une siccité après surpressage de 25 % environ au maximum. Les vinasses ne sont en général pas concentrées. R et G sont de l'ordre de :

|  | Sans déshydratation des pulpes | Avec déshydratation des pulpes |
|---|:---:|:---:|
| R | 0,2 | 0,18 |
| G | 940 | 1350 |

Les deux techniques ci-avant désignées, qui utilisent la séparation des phases soluble et insoluble des plantes à traiter, n'ont pour seul objet que cette séparation des constituants solubles et insolubles, à l'exclusion de la déshydratation simultanée maximum des parties insolubles des plantes.

De plus, ce type d'installation consomme beaucoup d'énergie, et dans des proportions qui excluent la possibilité de fonctionner en énergie totale, y compris le séchage de la phase insoluble, ou avec utilisation largement prépondérante de l'électricité.

Le procédé objet de la présente invention permet, avec l'utilisation combinée :

- d'un broyage très poussé de la matière première végétale,
- d'un traitement physico-chimique des matières glucidiques,
- d'une technique de séparation très efficace des phases soluble et insoluble dans un filtre-presse polyvalent, tel que celui faisant l'objet de la demande de brevet français n° 82 03766,
- de la technique connue de la recompression mécanique des vapeurs, d'obtenir les avantages spécifiques suivants :

- polyvalence de traitement dans la même installation, des plantes protéiques et glucidiques et des sous-produits végétaux humides, d'où un grand nombre possible d'heures de fonctionnement de cette installation, pendant la même période, ou à des périodes calendaires différentes :

- obtention d'une siccité des tourteaux supérieure à 45 %, voire 50 % selon les produits ;
- consommation réduite d'énergie primaire dans des proportions permettant un fonctionnement en énergie totale, à partir de diesels ou gazogènes, avec séchage des tourteaux.

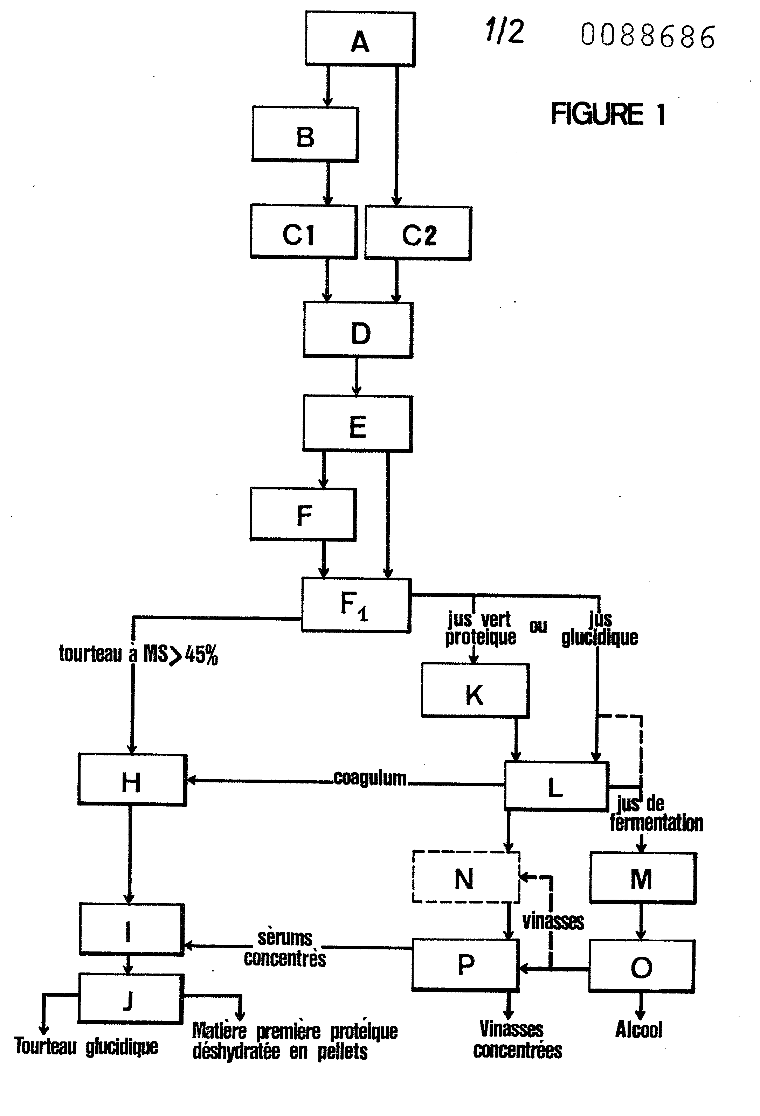

Le procédé selon l'invention comprend les étapes suivantes, représentées schématiquement dans le diagramme de la figure 1, ci-après :

Après alimentation A de la matière première végétale, et lavage B s'il s'agit de plantes glucidiques, on procède à une réduction en parties de section réduite en brins de 1 à 2 cm pour les plantes protéiques, $C_1$, ou en lamelles ou cossettes de 1 à 3 mm d'épaisseur pour les topinambours ou betteraves lavés, $C_2$.

Après écrasement, ou pré-éclatement D par broyage, on effectue un éclatement E le plus complet possible des cellules constituant le produit, permettant de libérer l'eau de constitution et les composants solubles qu'elle contient. Cet éclatement peut être notamment effectué par l'association d'aplatisseur et de broyeur à boulet de conception adaptée.

Pendant cette phase du procédé et pour les plantes glucidiques on ajoute des fibres végétales (paille, tourteaux de plantes fourragères, etc.) à raison de 0 à 10 % en poids des matières premières glucidiques, soit de 0 à 60 % en poids de la matière sèche contenue dans ces matières premières et on opère, selon un procédé décrit ci-après, un traitement physico-chimique F nécessaire pour favoriser la vitesse et le rendement de fermentation ultérieurs.

Puis, les plantes protéiques éclatées et les plantes glucidiques éclatées et traitées selon F subissent l'opération de filtration-pressage G dans un appareil assurant la progressivité de la concentration des matières insolubles, puis extrayant la proportion maximale de jus pour obtenir un tourteau de siccité très élevée, de 45 à 60 % de matière sèche.

Les jus séparés lors de l'opération de filtration-pressage subissent divers traitements :

. pour les jus protéiques, une coagulation thermique K et une séparation coagulum-sérum dans une décanteuse continue L, selon une technique connue ;

. éventuellement, pour les jus glucidiques, une décantation continue L, afin d'obtenir un jus avant fermentation exempt de matières coagulées en suspension.

Les jus glucidiques peuvent subir ensuite :

- une opération éventuelle de fermentation M et une opération éventuelle de distillation O, pour la production d'alcool, avec l'utilisation d'une récompression mécanique des vapeurs.

Après une osmose inverse N éventuelle, les jus protéiques subissent une opération de cencentration P des vinasses ou sérums, jusqu'à 50 % de matière sèche, voire 75 % selon les destinations des produits concentrés.

D'autre part, les tourteaux venant du pressage, après avoir été mélangés G au coagulum protéique, puis I aux sérums concentrés, sont soumis éventuellement à un séchage basse température J en circuit ouvert à air chaud, avec condensation partielle de la vapeur d'eau, ou en circuit fermé par pompe à chaleur à condensation totale de l'eau évaporée.

Le procédé de traitement physico-chimique F des plantes glucidiques est caractérisé en ce que, à la sortie du broyeur à boulets où s'effectue l'homogénéisation des matières glucidiques écrasées et des fibres végétales additionnées, le broyat est déversé dans un réacteur à l'intérieur duquel va se dérouler une série de réactions complexes destinées à favoriser le processus ultérieur d'extraction.

Ces réactions sont provoquées, d'une part, par addition de substances chimiques choisies en fonction de la composition des matières premières et, d'autre part, par l'application d'un traitement thermique mesuré, avec ou sans pression, traitement thermique favorisant le développement des réactions.

Les substances chimiques utilisées selon l'invention sont choisies de façon à provoquer à la fois une destructuration des parties solides, une coagulation des substances colloïdales et pectiques, et une dépolymérisation des composés à poids moléculaire élevé.

Parmi les produits chimiques utilisés, on citera d'une part les divers acides minéraux et organiques et d'autre part les bases minérales dosés de manière à obtenir une stabilisation chimique convenable et surtout une composition finale des produits correspondant aux exigences industrielles des traitements ultérieurs des uns et aux normes de l'alimentation animale des autres.

Les acides sont choisis de façon à amener le pH à une valeur de 4 à 1,5, qui favorise la transformation des fibres cellulosiques et provoque parfois une hydrolyse partielle.

Le traitement thermique consiste à chauffer l'ensemble des produits dans le réacteur à une température de 50 à 85°C, pendant une durée de 30 minutes à 2 heures. Les conditions du traitement thermique sont déterminées par la nature des glucides présents dans la matière première : Si l'on traite des produits contenant essentiellement des fructosanes, par exemple des topinambours, on ne peut dépasser 50°C afin de ne pas risquer de détruire le fructose. Par contre, si l'on traite des produits tels que la betterave, contenant essentiellement du saccharose, on peut augmenter la température jusqu'à 85°C, ce qui permettra de raccourcir la durée de la réaction.

La réaction étant terminée, on effectue un traitement chimique secondaire qui consiste en addition d'une base minérale pour ramener le pH aux environs de 4 pour les produits devant subir la fermentation ou aux environs de la neutralité pour les produits servant à l'alimentation du bétail.

Le traitement physico-chimique, tel qu'il est décrit ci-dessus, représente le processus de base. A partir de ce dernier, deux variantes sont possibles :

VARIANTE 1 - Combinaison du traitement physico-chimique atténué avec des actions enzymatiques spécifiques isolées ou associées. Dans ce cas, les conditions physico-chimiques du milieu sont adaptées de façon à correspondre à l'optimum d'action de l'enzyme ou des enzymes mises en oeuvre, ce qui conduit à moduler les dosages des produits chimiques d'addition ainsi que le niveau des températures en fonction de la composition des matières premières, d'une part, et des exigences propres aux préparations enzymatiques, d'autre part. Ces dernières agissent essentiellement sur la dépolymérisation des composés que traitement physico-chimique décrit prépare à l'action enzymatique. Sans cette préparation, cette action serait beaucoup moins efficace. Il est, en effet, connu que les composés végétaux, notamment les ligno-cellulosiques, sont difficilement accessibles aux conversions enzymatiques s'ils ne sont pas préalablement prédégradés.

L'accessibilité de ces composés est évidemment très variable, les uns étant très résistants, les autres beaucoup moins. Pour ces derniers, on a même réussi à limiter le conditionnement aux seules normes rhéologiques, ce qui a entrainé la variante 2, ci-dessous décrite.

VARIANTE 2 - Sous réserve d'une destructuration mécanique suffisante, selon les modalités décrites ci-dessus de certaines plantes, ces dernières peuvent être soumises aux seules actions enzymatiques avant la séparation des phases, obtenue par filtration-pressage. Cette variante s'étend alors au traitement des plantes protéiques. Elle détermine, entre autre, une diminution de la viscosité de la phase liquide en améliorant la filtrabilité.

Le traitement physico-chimique, portant sur l'ensemble de la matière première (substances végétales solides, substances végétales dissoutes plus fibres végétales additionnées), est un aspect capital du procédé. Il faut noter qu'il n'entraîne pas de dépense supplémentaire au niveau de l'énergie par rapport à la technique de diffusion, il peut même conduire à des économies quand il est inserré dans un processus intégré.

Par ailleurs, il permet d'obtenir après filtration-pressage des produits ainsi traités :

a) - des jus plus purs et moins oxydés que ceux issus des traitements classiques et qui, comme on l'a déjà indiqué, sont facilement filtrables, ce qui est loin d'être le cas général ;

b) - des tourteaux titrant entre 45 et 60 % de matière sèche, ce qui correspond à un taux d'extraction remarquable qui, pour une pression unique est de l'ordre de 75 à 95 %.

Ces tourteaux présentent, en outre, du point de vue alimentaire, des caractéristiques de richesse et de digestibilité accrues par rapport aux tourteaux produits par les méthodes classiques.

Un autre avantage du traitement physico-chimique ci-dessus est le suivant :

Les broyats additionnés de fibres cellulosiques contiennent des polysaccharides qui, dans les procédés d'extraction classiques des glucides, se retrouvent inaltérés dans la phase solide. Au contraire, dans le procédé objet de l'invention les chaînes linéaires glucosiques sont partiellement rompues avec production de glucides qui s'ajoutent à ceux que les plantes contiennent sous forme extractible, si bien que la quantité de glucides produite est supérieure à la quantité de glucides entrée, déterminée par l'analyse des extractibles. On exploite donc, en plus des glucides normalement extraits par les méthodes connues, une partie des glucides contenus en puissance dans les substances cellulosiques.

Pour illustrer une des applications concrètes du procédé de traitement physico-chimique des plantes glucidiques on citera comme exemple non limitatif, le traitement des betteraves en distillerie. A partir des racines de ces plantes (ou éventuellement des plantes entières) on obtient, d'une part, des jus destinés à la fermentation, d'autre part, des tourteaux destinés à l'alimentation du bétail .
Mode opératoire :

Les racines lavées, découpées et broyées sont introduites dans un réacteur pourvu d'un système de chauffage, d'un agitateur et d'organes de contrôle (thermomètre, manomètre, pH mètre) ; un tube d'adduction permet d'introduire de l'air comprimé. Le pH est réglé à 2,2 par addition d'acide sulfurique à 66°B. Après chauffage pendant 1 heure 30 minutes à 80°C, sous une pression de 3 bars, avec agitation, on neutralise à chaud soit avec une solution aqueuse d'ammoniaque à 22°, soit avec de l'ammoniac anhydre, pour amener le pH à 4. La masse ainsi traitée est dirigée vers le poste de filtration-pressage après refroidissement ou échange de chaleur.

Ainsi, une tonne de broyat de betteraves, ayant une richesse de 21 % de matière sèche totale et 16 % de glucides extractibles, a donné, avec une addition de 30 kg de fibres déshydratées de luzerne, plus 500 kg d'eau :
- 1390 kg de jus à 12,15 % de matière sèche soit 168,90 kg de matière sèche

140 kg de tourteau    à 50,83 % de matière sèche soit   71,10 kg de   "      "
-----------
Total       240,- kg

Le bilan des glucides est le suivant :

- 1390 kg de jus à 11,97 % de glucides totaux     166,38 kg
- 140 kg de tourteau contenant :

    36 kg de matière sèche non glucidique

    27 kg de matière sèche provenant des fibres

    7 kg de glucides totaux                      7,00

    70     d'eau

                   Total           173,38 kg de glucides

        Entrée glucides extractibles      160,-    kg

        Excédent                   13,38 kg

soit un gain de 8,36 %.

Ainsi, le procédé selon l'invention a pour objet de permettre un transfert maximum de l'eau à extraire, sous forme liquide dans les jus, afin de permettre, par recompression mécanique de vapeur, d'en assurer l'extraction au moindre coût énergétique (environ 20 kWh/t d'eau. Ceci suppose la séparation maximale des parties soluble et insoluble contenues dans les plantes à traiter, ce qui est réalisé notamment par le broyage des cellules elles-mêmes, et par le traitement physico-chimique des plantes glucidiques, avant l'opération de filtration-pressage.

De plus, le procédé permet de supprimer l'installation de déshydratation classique haute température, ainsi que le broyage des produits secs, puisque ceux-ci sont broyés en phase humide et que la finition de séchage ne requiert qu'une petite installation de séchage basse température.

A l'opposé, le procédé antérieur selon le brevet n°2.294.647 avait pour objet principal d'obtenir des concentrés protéiques riches, ce qui est nécessairement au détriment de l'efficacité de séparation des phases et qui ne conduit pas à une simplification des chaînes d'équipement.

Le procédé de traitement des plantes protéiques ou glucidiques, tel que schématisé dans la figure 1, permet un fonctionnement en énergie totale avec l'utilisation exclusive d'un moteur thermique de rendement

$$R = \frac{\text{Energie mécanique produite}}{\text{Energie thermique consommée}} = 0,3 \text{ à } 0,4$$

et l'obtention de produits finis à 88 % de matière sèche à partir de matières premières à 20 % de matière sèche. La consommation énergétique globale G pour ce procédé est comprise entre 500 et 1000.

A partir de matières premières protéiques, à 20 % de matière sèche, le procédé de transformation, sans fermentation ni distillation, pour récupérer l'ensemble des matières sèches sous forme de tourteaux à 88 % de matière sèche, permet d'utiliser l'énergie électrique avec une consommation de 130 à 200 kWh par tonne de produit sec, à laquelle s'ajoute la consommation calorique de la phase de coagulation thermique, et de la finition de séchage.

A partir de matières premières glucidiques, à 18-22 % de matière sèche et 14 à 17 % de sucres fermentescibles, sans concentration des vinasses, le procédé selon l'invention permet, après fermentation et distillation des jus, de produire de l'éthanol à 92 % et des tourteaux déshydratés à 87-90 % de matière sèche, avec une consommation énergétique globale G de 450 à 700. Avec une concentration des vinasses jusqu'à 75 % de matière sèche, la consommation globale G est de 500 à 850.

EXEMPLE 1 : Traitement de la luzerne à 20 % de matière sèche

La séparation mécanique G des phases soluble et insoluble conduit à la répartition suivante :

- jus                3450 kg dont 405 kg matière sèche soluble
- tourteaux           950 kg dont 475 kg matière sèche (50 % de M.S.)
                     _____      ____

matière première     4400 kg dont 880 kg matière sèche

La coagulation des protéines K conduit à la séparation suivante sur les jus :

- sérum     3090 kg dont    243 kg matière sèche
- coagulum    360 kg dont    162 kg matière sèche jus       3450 kg dont    405 kg matière sèche

Après concentration P du sérum et finition du séchage H et J des matières solides on obtient :

- tourteau à 88 % de matière sèche (provenant du tourteau G et du sérum K)

   . concentré sérum )
                 ) = 816 kg et ( 2766 kg eau évaporée en recompression de vapeur (RMV)
   . tourteau séché )
                 )        ( 458 kg eau évaporée en séchage basse température (SBT)

- coagulum à 88 % M.S. = 184 kg     176 kg eau évaporée en séchage basse température (SBT)

                    1000 kg       2766 kg eau évaporée en RMV
                              + 634 kg eau évaporée en SBT

81 % de l'eau est éliminée par RMV.

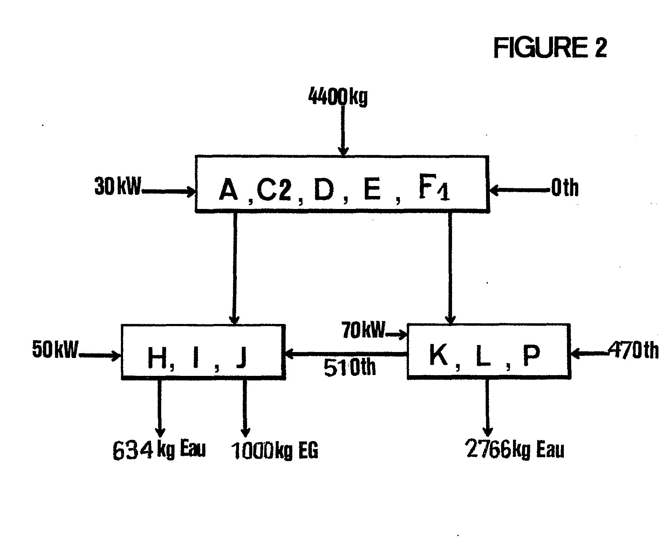

La consommation d'énergie thermique (th) et électrique (KW) est représentée schématiquement dans la figure 2 ; on en déduit R = 0,41

$$G = \frac{150 \times 2,2 + 470}{0,88} = 909$$

dans le cas où tous les produits sont séchés, avec l'utilisation d'énergie thermique classique pour la finition du séchage.

L'exemple 1 montre que la plus grande partie de l'eau (81 %) est évacuée sous forme liquide, par RMV, ce qui permet d'obtenir des économies très importantes d'énergie, de l'ordre de 64 % (par rapport à la déshydratation pour laquelle G = 2300).

Pour le topinambour ou la betterave, à 20 % de matière sèche et 15 % de sucres fermentescibles, et dans le cas de la distillation sans concentration des vinasses, avec production d'éthanol à 92 % d'alcool pur et de tourteaux à 88 % de matière sèche, on note les économies suivantes :

|   | Distillation classique | Distillation selon l'invention | Economie par procédé selon l'invention |
|---|---|---|---|
| R | 0,18 | 0,6 | |
| G | 1350 | 510 | 62 % |

Dans le cas ci-dessus, avec concentration des vinasses à 75 % de matière sèche, la consommation globale est de 600 environ.

Le dimensionnement des appareillages est prévu pour s'adapter aux différents produits à traiter, en particulier pour ce qui concerne la concentration des sérums ou vinasses. Pour certains cas d'application du procédé, selon les quantités relatives de produits à traiter, il est prévu de compenser les différences de besoins en débit en RMV, par une installation d'osmose inverse effectuant une préconcentration des liquides.

L'opération éventuelle de séchage basse température est effectuée en utilisant notamment la chaleur récupérée sur l'eau de condensation et le traitement physicochimique.

R E V E N D I C A T I O N S

1.      Procédé de traitement de matières végétales protéiques et glucidiques et de sous-produits végétaux contenant de 60 à 90 % d'eau, pour récupérer, d'une part, tout ou partie des constituants sous forme de tourteaux déshydratés, et d'autre part, des jus glucidiques destinés à la fermentation et à la distillation, caractérisé en ce qu'il comporte les étapes suivantes, effectuées au moindre coût énergétique et dans la même installation pour les matières protéiques et glucidiques :

a) broyage très poussé de la matière première jusqu'à l'éclatement des cellules végétales, obtenu par l'association d'un aplatisseur et d'un broyeur à boulet ;

b) filtration-pression dans un appareil assurant une concentration progressive des matières insolubles, de façon à séparer la quantité maximale d'eau dans les jus et obtenir un tourteau dont la siccité est de 45 à 60 % de matière sèche ;

c) élimination de l'eau des jus par recompression mécanique des vapeurs et séchage des tourteaux à basse température pour obtenir finalement des tourteaux à 87-90 % de matière sèche.

2.      Procédé selon la revendication 1, caractérisé en ce que les matières glucidiques sont mélangées à des fibres végétales avant le broyage poussé et sont soumises, préalablement à la filtration-pression, à un traitement physico-chimique destiné à favoriser la vitesse et le rendement de fermentation ultérieurs.

3.      Procédé selon la revendication 2, caractérisé en ce qu'on mélange à la matière végétale de 0 à 10 % de fibres végétales, avant broyage et homogénéisation, qu'on ajoute au broyat obtenu un acide minéral ou organique de façon à amener le pH entre 4 et 1,5 ; qu'on chauffe ensuite le mélange obtenu entre 50 et 85° C pendant 30 min. à 2 h et qu'on ramène ensuite, par addition d'une base minérale, le pH à une valeur appropriée aux opérations d'extraction ultérieure et compatible avec les normes auxquelles les produits alimentaires finalement obtenus doivent se conformer.

4.      Procédé selon la revendication 1, caractérisé en ce que les jus protéiques recueillis par filtration-pression sont soumis à une coagulation thermique puis à une décantation qui permet de séparer un coagulum et un sérum qui est ensuite concentré jusqu'à 50-75 % de matière sèche, coagulum et sérum concentré étant réunis au tourteau de pressage avant la finition de séchage.

5.      Procédé selon l'une des revendications 1 à 4, caractérisé en ce qu'on préconcentre par osmose inverse les sérums ou vinasses pour compenser les variations de taux de matières sèches de ceux-ci selon la nature des matières premières, afin d'adapter les régimes de fonctionnement de la concentration en RMV à un taux constant de matière sèche.

6.      Procédé selon l'une des revendications 1 à 5, caractérisé en ce que la finition de séchage des tourteaux est effectuée à basse température, en circuit ouvert à air chaud avec condensation partielle de la vapeur d'eau, ou en circuit fermé par pompe à chaleur avec condensation totale de l'eau évaporée.

7.      Procédé selon l'une des revendications 1 à 6, pour le traitement des plantes protéiques ou glucidiques, caractérisé en ce qu'il permet un fonctionnement en énergie totale avec l'utilisation exclusive d'un moteur thermique de rendement R = 0,3 à 0,4 et l'obtention de produits finis à 88 % de matière sèche à partir de matière première à 20 % de matière sèche, avec une consommation énergétique globale comprise entre 500 et 1000.

8.      Procédé selon l'une des revendications 1 à 6, caractérisé en ce que pour transformer des matières premières protéiques à 20 % de matière sèche sans fermentation ni distillation, afin de récupérer l'ensemble des matières sèches sous forme de tourteaux à 88 % de matière sèche, on utilise l'énergie électrique avec une consommation de 130 à 200 kWh par tonne de produit sec obtenu, à laquelle s'ajoute la consommation calorique de la phase de coagulation thermique et celle de la finition de séchage.

9.      Procédé selon l'une des revendications 1 à 3, caractérisé en ce que les jus glucidiques recueillis par filtration-pressage sont décantés, soumis à fermentation et distillation, avec utilisation d'une recompression mécanique des vapeurs, pour la production d'éthanol à 92 % et de tourteaux à 87-90 % de matière sèche, la consommation énergétique totale G étant de 450 à 700 en partant de matières végétales glucidiques à 18-22 % de matière sèche et 14 à 17 % de sucres fermentescibles.

10.    Procédé selon la revendication 9, caractérisé en ce que les vinasses étant concentrées jusqu'à 75 % de matière sèche, la consommation globale G est de 500 à 850.

FIGURE 1

**FIGURE 2**

**Office européen
des brevets**

**RAPPORT DE RECHERCHE EUROPEENNE**

0088686
Numéro de la demande

EP 83 40 0439

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | FR-A-2 473 271 (AGROPROTEINE IND) *Résumé 1,9,10; page 1, lignes 1-8,33-38; page 2, lignes 32-36; page 3, lignes 1-17* | 1 | A 23 J 1/00 C 12 C 5/00 C 13 D 1/08 C 13 L 3/00 |
| A | | 6 | |
| | --- | | |
| Y | FR-A-1 079 700 (SOCIETE NORMAND D'EXPLOITATION AGRICOLE) *Résumé 1,2* | 1 | |
| | --- | | |
| Y | FR-A-2 148 157 (BATLEY-JANNS ENTERPRISES ET BATLEY) *Résumé 1,2,4; page 6, lignes 26-30* | 1 | |
| A | | 4 | DOMAINES TECHNIQUES RECHERCHES (Int. Cl. ³) |
| | --- | | A 23 J C 12 C C 13 D C 13 L C 08 B |
| Y | FR-A-2 248 321 (CASTILLIO) *Résumé 1* | 1,9 | |
| | --- | | |
| Y | FR-A- 921 566 (J.CATINEAU et al) *Résumé 1* | 1,9 | |
| | --- | | |
| A | DE-A-2 906 977 (A.BALLWEG) *Revendications 1,2; page 9, lignes 10-28* | 1,3 | |
| | --- -/- | | |

Le présent rapport de recherche a été établi pour toutes les revendications

| Lieu de la recherche LA HAYE | Date d'achèvement de la recherche 13-06-1983 | Examinateur PEETERS J.C. |
|---|---|---|

## RAPPORT DE RECHERCHE EUROPEENNE

**Office européen des brevets**

| DOCUMENTS CONSIDERES COMME PERTINENTS | | | Page 2 |
|---|---|---|---|
| Catégorie | Citation du document avec indication, en cas de besoin, des parties pertinentes | Revendication concernée | CLASSEMENT DE LA DEMANDE (Int. Cl. ³) |
| A | BE-A- 444 896 (W.FISCHER) *Résumé* | 1,3 | |
| A | FR-A- 397 672 *En entier* | 1,3 | |
| A | FR-A-1 032 331 (M.MOREAU) *Résumé; page 1, colonne 2, alinéa 3* | 1,3 | |
| A | DE-C- 204 197 (C.STEFFEN) *Revendications 1,2* | 1,3 | |
| | | | DOMAINES TECHNIQUES RECHERCHES (Int. Cl. ³) |

Le présent rapport de recherche a été établi pour toutes les revendications

| Lieu de la recherche | Date d'achèvement de la recherche | Examinateur |
|---|---|---|
| LA HAYE- | 13-06-1983 | PEETERS J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

   & : membre de la même famille, document correspondant

OEB Form 1503 03 82